# EUROPEAN PATENT APPLICATION

(11) **EP 1 020 437 A1**
(43) Date of publication of application: **19.07.2000**
(21) Application number: 98911045.7
(22) Date of filing: 26.03.1998
(51) Int. Cl.: C07C 311/02, C07C 303/38, C07C 309/80, C07C 303/02

(54) **PROCESS FOR PRODUCING HALOGENOALKYLSULFONAMIDE DERIVATIVES**

(30) Priority: 26.03.1997 JP 9315997
(71) Applicant: Azwell Inc., Osaka 540-8575 (JP)
(72) Inventor: KASHIMA, Kenichi, Fujiidera-shi, Osaka 583-0016 (JP); SAKAMOTO, Yasuhiko, Habikino-shi, Osaka 583-0882 (JP); TAKEMURA, Shigetaka, Riverside Shirokita 35-610, Osaka-shi, Osaka 534-0001 (JP)
(74) Representative: Schüssler, Andrea, Dr.
(86) International application number: JP9801378
(87) International publication number: WO9842660

(57) **Abstract**

A process for preparing a halogenoalkylsulfonamide derivative represented by the general formula (I): 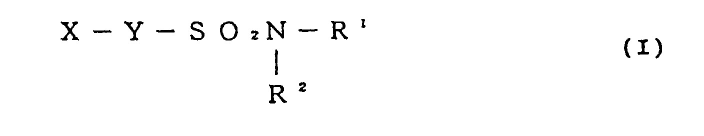 wherein R¹ is alkyl, hydroxyalkyl, cycloalkyl, or phenyl which may have alkyl, alkoxy, halogen, hydroxyl, trifluoromethyl, nitro and amino; R² is hydrogen atom, alkyl, hydroxyalkyl, cycloalkyl, or phenyl which may have alkyl, alkoxy, halogen, hydroxyl, trifluoromethyl, nitro and amino; X is chlorine, bromine or iodine; and Y is alkylene group having 5 to 12 carbon atoms; an intermediate; and a process for preparing the intermediate. The amide derivative is a useful compound as an intermediate for preparing a diazacycloalkanealkylsulfonamide derivative, which has antiallergic activity and thereby is useful as a medicament for preventing and treating diseases such as bronchial asthma, allergic rhinitis, atopic dermatitis, urticaria, and the like.

## Description

### TECHNICAL FIELD

The present invention relates to a process for preparing a halogenoalkylsulfonamide derivative. More specifically, the present invention relates to a process for preparing a halogenoalkylsulfonamide derivative which is useful as an intermediate for preparing a diazacycloalkanealkylsulfonamide derivative, which has excellent antiallergic activity and thereby is useful as a medicament for preventing and treating diseases such as bronchial asthma, allergic rhinitis, atopic dermatitis, urticaria and the like, and to a halogenoalkylsulfonyl chloride which is useful as an intermediate for preparing the halogenoalkylsulfonamide derivative, and a process for preparing the same.

### BACKGROUND ART

The halogenoalkylsulfonamide derivative of which alkylene group of the main chain has not less than 5 carbon atoms is a useful intermediate for a diazacycloalkanealkylsulfonamide derivative which has been used as an antiallergic agent. As a process for preparing the halogenoalkylsulfonamide derivative, there have been known processes comprising reacting a halogenoalkylsulfonyl chloride with an amine [*Liebigs Ann*. *Chem*., **635**, 91 (1960); *J. Org. Chem*., **33**, 3066 (1968); Japanese Patent Laid-Open No. 17996/1995; WO96/03375 Pamphlet (1996)].

However, since all of these processes require to use relatively expensive amines in excessive amounts to the halogenoalkylsulfonyl chloride, there has been desired to establish a process in which the reduction in the amount of the amines can be achieved.

An object of the present invention is to provide a process capable of preparing economically and industrially advantageously a halogenoalkylsulfonamide derivative, of which alkylene group of the main chain has 5 to 12 carbon atoms.

Another object of the present invention is to provide a halogenoalkylsulfonyl chloride which is an intermediate useful for preparing the halogenoalkylsulfonamide derivative, and a process for preparing the same.

These and other objects of the present invention will be apparent from the following description.

### DISCLOSURE OF INVENTION

According to the present invention, there are provided:
[1] a process for preparing a halogenoalkylsulfonamide derivative represented by the general formula (Ia): 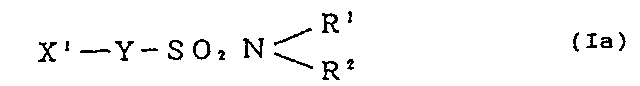 wherein R¹ is a straight or branched chain alkyl group having 1 to 6 carbon atoms, a hydroxyalkyl group having 1 to 4 carbon atoms, a cycloalkyl group having 3 to 8 carbon atoms, or a phenyl group which may have on its phenyl ring 1 to 3 substituents selected from the group consisting of an alkyl group having 1 to 4 carbon atoms, an alkoxy group having 1 to 4 carbon atoms, a halogen atom, hydroxyl group, trifluoromethyl group, nitro group and amino group; R² is hydrogen atom, a straight or branched chain alkyl group having 1 to 6 carbon atoms, a hydroxyalkyl group having 1 to 4 carbon atoms, a cycloalkyl group having 3 to 8 carbon atoms, or a phenyl group which may have on its phenyl ring 1 to 3 substituents selected from the group consisting of an alkyl group having 1 to 4 carbon atoms, an alkoxy group having 1 to 4 carbon atoms, a halogen atom, hydroxyl group, trifluoromethyl group, nitro group and amino group; X¹ is chlorine atom or bromine atom; and Y is an alkylene group having 5 to 12 carbon atoms, characterized by reacting a halogenoalkylsulfonyl chloride represented by the general formula (II):

   X¹-Y-SO₂Cl (II)

   wherein X¹ and Y are as defined above, with
   an amine represented by the general formula (III): 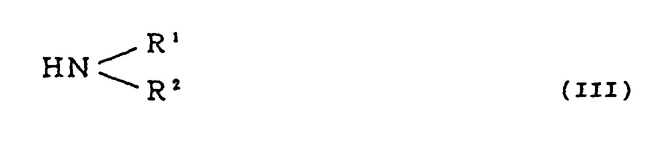 wherein R¹ and R² are as defined above;
[2] a halogenoalkylsulfonyl chloride represented by the general formula (IIa):

   Br-Y-SO₂Cl (IIa)

   wherein Y is an alkylene group having 5 to 12 carbon atoms; and
[3] a process for preparing a halogenoalkylsulfonyl chloride represented by the general formula (IIa):

   Br-Y-SO₂Cl (IIa)

   wherein Y is an alkylene group having 5 to 12 carbon atoms,
   characterized by reacting a sodium bromoalkylsulfonate represented by the general formula (IV):

   Br-Y-SO₃Na (IV)

   wherein Y is as defined above,
   with a chlorinating agent.

### BEST MODE FOR CARRYING OUT THE INVENTION

The halogenoalkylsulfonamide derivative is represented by the general formula (I): 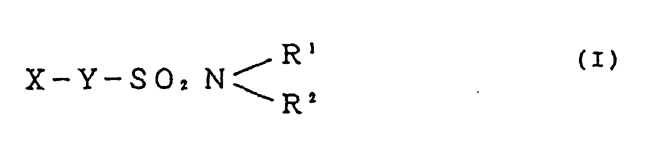 wherein R¹ is a straight or branched chain alkyl group having 1 to 6 carbon atoms, a hydroxyalkyl group having 1 to 4 carbon atoms, a cycloalkyl group having 3 to 8 carbon atoms, or a phenyl group which may have on its phenyl ring 1 to 3 substituents selected from the group consisting of an alkyl group having 1 to 4 carbon atoms, an alkoxy group having 1 to 4 carbon atoms, a halogen atom, hydroxyl group, trifluoromethyl group, nitro group and amino group; R² is hydrogen atom, a straight or branched chain alkyl group having 1 to 6 carbon atoms, a hydroxyalkyl group having 1 to 4 carbon atoms, a cycloalkyl group having 3 to 8 carbon atoms, a phenyl group which may have on its phenyl ring 1 to 3 substituents selected from the group consisting of an alkyl group having 1 to 4 carbon atoms, an alkoxy group having 1 to 4 carbon atoms, a halogen atom, hydroxyl group, trifluoromethyl group, nitro group and amino group; X is chlorine atom, bromine atom or iodine atom; and Y is an alkylene group having 5 to 12 carbon atoms, which is a useful compound as an intermediate for preparing a diazacycloalkanealkylsulfonamide derivative.

In R¹ and R², the straight or branched chain alkyl group having 1 to 6 carbon atoms includes methyl group, ethyl group, n-propyl group, isopropyl group, n-butyl group, isobutyl group, sec-butyl group, tert-butyl group, n-pentyl group, isopentyl group, sec-pentyl group, neopentyl group, tert-pentyl group, n-hexyl group, isohexyl group, sec-hexyl group, neohexyl group and tert-hexyl group. The hydroxyalkyl group having 1 to 4 carbon atoms includes hydroxymethyl group, hydroxyethyl group, hydroxypropyl group and hydroxybutyl group. The cycloalkyl group having 3 to 8 carbon atoms includes cyclopropyl group, cyclobutyl group, cyclopentyl group, cyclohexyl group, cycloheptyl group and cyclooctyl group. In the phenyl group which may have on its phenyl ring 1 to 3 substituents selected from the group consisting of an alkyl group having 1 to 4 carbon atoms, an alkoxy group having 1 to 4 carbon atoms, a halogen atom, hydroxyl group, trifluoromethyl group, nitro group and amino group, the alkyl group having 1 to 4 carbon atoms includes methyl group, ethyl group, n-propyl group, isopropyl group, n-butyl group, isobutyl group, sec-butyl group and tert-butyl group. The alkoxy group having 1 to 4 carbon atoms includes methoxy group, ethoxy group, propoxy group and butoxy group. The halogen atom includes fluorine atom, chlorine atom, bromine atom and iodine atom.

Among R¹, the alkyl group having 1 to 6 carbon atoms, the hydroxyalkyl group having 1 to 4 carbon atoms and the cycloalkyl group having 3 to 8 carbon atoms are preferable, and the hydroxyalkyl group having 1 to 4 carbon atoms and the cycloalkyl group having 3 to 8 carbon atoms are more preferable. In addition, among the cycloalkyl group having 3 to 8 carbon atoms, a cycloalkyl group having 3 to 6 carbon atoms is preferable, and cyclopropyl group and cyclobutyl group are particularly preferable.

Among R², hydrogen atom, a straight chain alkyl group having 1 to 6 carbon atoms, a hydroxyalkyl group having 1 to 4 carbon atoms and a cycloalkyl group having 3 to 6 carbon atoms are preferable, and hydrogen atom is particularly preferable.

In the present invention, a particularly preferable combination of R¹ and R² is where R¹ is cyclopropyl group, cyclobutyl group or a hydroxyalkyl group having 1 to 4 carbon atoms and R² is hydrogen atom, from the viewpoint of preparing a pharmacologically effective diazacycloalkanealkylsulfonamide derivative.

As described above, X is chlorine atom, bromine atom or iodine atom.

As described above, Y is an alkylene group having 5 to 12 carbon atoms, preferably 5 to 8 carbon atoms, which may be a straight or branched chain.

In addition, among the halogenoalkylsulfonamide derivatives represented by the general formula (I), from the viewpoint of preparing a pharmacologically effective diazacycloalkanealkylsulfonamide derivative, preferable is a halogenoalkylsulfonamide derivative wherein R¹ is a straight or branched chain alkyl group having 1 to 6 carbon atoms, a cycloalkyl group having 3 to 6 carbon atoms or a hydroxyalkyl group having 1 to 4 carbon atoms; R² is hydrogen atom, a straight chain alkyl group having 1 to 6 carbon atoms, a hydroxyalkyl group having 1 to 4 carbon atoms or a cycloalkyl group having 3 to 6 carbon atoms; X is chlorine atom, bromine atom or iodine atom; and Y is an alkylene group having 5 to 12 carbon atoms. More preferable is a halogenoalkylsulfonamide derivative wherein R¹ is a cycloalkyl group having 3 to 6 carbon atoms or a hydroxyalkyl group having 1 to 4 carbon atoms; R² is hydrogen atom; X is chlorine atom, bromine atom or iodine atom; and Y is an alkylene group having 5 to 12 carbon atoms. Still more preferable is a halogenoalkylsulfonamide derivative wherein R¹ is cyclopropyl group, cyclobutyl group or a hydroxyalkyl group having 1 to 4 carbon atoms; R² is hydrogen atom; X is chlorine atom, bromine atom or iodine atom; and Y is an alkylene group having 5 to 12 carbon atoms. Particularly preferable is a halogenoalkylsulfonamide derivative wherein R¹ is cyclopropyl group, cyclobutyl group or a hydroxyalkyl group having 1 to 4 carbon atoms; R² is hydrogen atom; X is chlorine atom, bromine atom or iodine atom; and Y is an alkylene group having 5 to 8 carbon atoms.

As to the processes for preparing the halogenoalkylsulfonamide derivative represented by the general formula (I), the process for preparing a halogenoalkylsulfonamide derivative represented by the general formula (Ia): 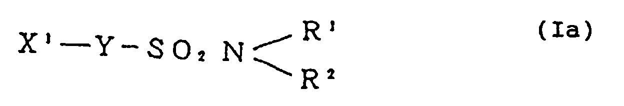 wherein R¹, R² and Y are as defined above; and X¹ is chlorine atom or bromine atom,
which is a compound wherein X is chlorine atom or bromine atom in the general formula (I) is different from that for preparing a halogenoalkylsulfonamide derivative represented by the general formula (Ib): 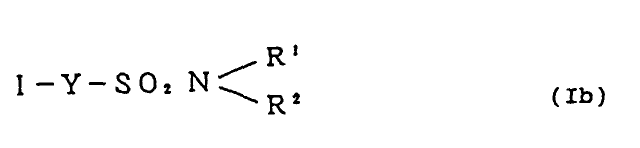 wherein R¹, R² and Y are as defined above,
which is a compound wherein X is iodine atom in the general formula (I).

First, the process for preparing the halogenoalkylsulfonamide derivative represented by the general formula (Ia) will be described.

The halogenoalkylsulfonamide derivative represented by the general formula (Ia) can be obtained by reacting a halogenoalkylsulfonyl chloride represented by the general formula (II):

X¹-Y-SO₂Cl (II)

wherein X¹ and Y are as defined above, with
an amine represented by the general formula (III): 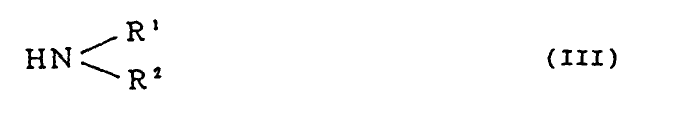 wherein R¹ and R² are as defined above.

As the halogenoalkylsulfonyl chloride represented by the general formula (II), there can be cited a halogenoalkylsulfonyl chloride represented by the general formula (IIa):

Br-Y-SO₂Cl (IIa)

wherein Y is as defined above; and a halogenoalkylsulfonyl chloride represented by the general formula (IIb):

Cl-Y-SO₂Cl (IIb)

herein Y is as defined above.

The halogenoalkylsulfonyl chloride represented by the general formula (IIa) is concretely a bromoalkylsulfonyl chloride.

Representative examples of the halogenoalkylsulfonyl chloride represented by the general formula (IIa) include 5-bromopentanesulfonyl chloride, 6-bromohexanesulfonyl chloride, 7-bromoheptanesulfonyl chloride, 8-bromooctanesulfonyl chloride, 2-bromohexanesulfonyl chloride, 3-bromohexanesulfonyl chloride, 5-bromohexanesulfonyl chloride, 9-bromononanesulfonyl chloride, 10-bromodecanesulfonyl chloride, 11-bromoundecanesulfonyl chloride and 12-bromododecanesulfonyl chloride.

In the general formula (IIa), it is preferable that Y is an alkylene group having 5 to 8 carbon atoms.

The process for preparing the halogenoalkylsulfonyl chloride represented by the general formula (IIa) will be described in detail in a later section.

The halogenoalkylsulfonyl chloride represented by the general formula (IIb) is concretely a chloroalkylsulfonyl chloride.

Representative examples of the halogenoalkylsulfonyl chloride represented by the general formula (IIb) include 5-chloropentanesulfonyl chloride, 6-chlorohexanesulfonyl chloride, 7-chloroheptanesulfonyl chloride, 8-chlorooctanesulfonyl chloride, 2-chlorohexanesulfonyl chloride, 3-chlorohexanesulfonyl chloride, 5-chlorohexanesulfonyl chloride, 9-chlorononanesulfonyl chloride, 10-chlorodecanesulfonyl chloride, 11-chloroundecanesulfonyl chloride and 12-chlorododecanesulfonyl chloride.

In the general formula (IIb), it is preferable that Y is an alkylene group having 5 to 8 carbon atoms.

In the amine represented by the general formula (III), each of R¹ and R² is the same as R¹ and R² in the halogenoalkylsulfonamide derivative represented by the general formula (I).

Among the amines represented by the general formula (III), from the viewpoint of obtaining a desired halogenoalkylsulfonamide derivative represented by the general formula (I), preferable is an amine wherein R¹ is a straight or branched chain alkyl group having 1 to 6 carbon atoms, a cycloalkyl group having 3 to 6 carbon atoms or a hydroxyalkyl group having 1 to 4 carbon atoms; and R² is hydrogen atom, a straight chain alkyl group having 1 to 6 carbon atoms, a hydroxyalkyl group having 1 to 4 carbon atoms or a cycloalkyl group having 3 to 6 carbon atoms. More preferable is an amine wherein R¹ is a cycloalkyl group having 3 to 6 carbon atoms or a hydroxyalkyl group having 1 to 4 carbon atoms; and R² is hydrogen atom. Particularly preferable is an amine wherein R¹ is cyclopropyl group, cyclobutyl group or a hydroxyalkyl group having 1 to 4 carbon atoms; and R² is hydrogen atom.

Therefore, in the amine represented by the general formula (III), preferable are primary amines such as methylamine, ethylamine, n-propylamine, isopropylamine, n-butylamine, isobutylamine, sec-butylamine, tert-butylamine, n-pentylamine, isopentylamine, sec-pentylamine, neopentylamine, tert-pentylamine, n-hexylamine, isohexylamine, sec-hexylamine, neohexylamine, tert-hexylamine, cyclopropylamine, cyclobutylamine, cyclopentylamine, cyclohexylamine, hydroxymethylamine, hydroxyethylamine, hydroxypropylamine, hydroxybutylamine and aniline; and secondary amines such as dimethylamine, diethylamine, dipropylamine and dibutylamine. More preferable are cyclopropylamine, cyclobutylamine, cyclopentylamine, cyclohexylamine, hydroxyethylamine, hydroxypropylamine and hydroxybutylamine. Still more preferable are cyclopropylamine, cyclobutylamine, hydroxyethylamine and hydroxypropylamine. Particularly preferable are cyclopropylamine, hydroxyethylamine and hydroxypropylamine.

In the present invention, particularly preferred amines are those amines in the general formula (III), wherein R¹ is a hydroxyalkyl group having 1 to 4 carbon atoms or a cycloalkyl group having 3 to 8 carbon atoms; and R² is hydrogen atom, a straight or branched chain alkyl group having 1 to 6 carbon atoms, a hydroxyalkyl group having 1 to 4 carbon atoms, a cycloalkyl group having 3 to 8 carbon atoms, a phenyl group which may have on its phenyl ring 1 to 3 substituents selected from the group consisting of an alkyl group having 1 to 4 carbon atoms, an alkoxy group having 1 to 4 carbon atoms, a halogen atom, hydroxyl group, trifluoromethyl group, nitro group and amino group.

As described above, the halogenoalkylsulfonamide derivative represented by the general formula (Ia) can be obtained by reacting the halogenoalkylsulfonyl chloride represented by the general formula (II) with the amine represented by the general formula (III).

It is preferable that the amount of the amine represented by the general formula (III) is generally 2 to 4 mol per one mol of the halogenoalkylsulfonyl chloride when the reaction is carried out in the absence of an inorganic base, and that the amount of the amine is 1 to 3 mol per one mol of the halogenoalkylsulfonyl chloride when the reaction is carried out in the presence of an inorganic base.

More strictly, when the reaction is carried out in the absence of an inorganic base, the amount of the amine differs depending upon the kinds of the amines. When the amine has one hydroxyalkyl group, it is desired that the amount of the amine is 2 to 3.3 mol per one mol of the halogenoalkylsulfonyl chloride. When the amine has two hydroxyalkyl groups, it is desired that the amount of the amine is 2 to 4 mol per one mol of the halogenoalkylsulfonyl chloride. In addition, when the amine has no hydroxyalkyl group, it is desired that the amount of the amine is 2 to 2.2 mol per one mol of the halogenoalkylsulfonyl chloride.

When the reaction is carried out in the presence of an inorganic base, the inorganic base includes, for instance, alkali metal hydroxides such as lithium hydroxide, sodium hydroxide and potassium hydroxide, sodium hydrogencarbonate, potassium hydrogencarbonate, sodium carbonate, potassium carbonate, and the like. Among them, alkali metal hydroxides such as lithium hydroxide, sodium hydroxide and potassium hydroxide are preferable.

It is desired that the amount of the inorganic base is not less than one mol per one mol of the halogenoalkylsulfonyl chloride, from the viewpoint of sufficient reduction of the amount of the amine, and that the amount of the inorganic base is at most 1.5 mol, preferably at most 1.2 mol, from the viewpoint of avoidance of the influence of hydrolysis.

When the inorganic base is used in the above amount, the amount of the amine differs depending upon the kinds of the amines. When the amine has one hydroxyalkyl group, it is desired that the amount of the amine is 1 to 1.5 mol per one mol of the halogenoalkylsulfonyl chloride. When the amine has two hydroxyalkyl groups, it is desired that the amount of the amine is 1 to 3 mol per one mol of the halogenoalkylsulfonyl chloride. In addition, when the amine has no hydroxyalkyl group, it is desired that the amount of the amine is 1 to 1.2 mol per one mol of the halogenoalkylsulfonyl chloride.

In the present invention, in the course of reaction, when the reaction is carried out in the presence of the inorganic base as described above, there is an advantage that the amount of the relatively expensive amine represented by the general formula (III) can be reduced. Furthermore, since the inorganic base is used, the resulting product can be easily separated from the inorganic base after the conclusion of the reaction. Accordingly, there is an advantage that complicated procedures as the case where an organic base is used are not required.

In the present invention, an organic base, such as pyridine, triethylamine, diisobutylethylamine, N-ethyldiisopropylamine, 4-N,N-dimethylaminopyridine, 1,8-diazabicyclo[5,4,0]-7-undecene or 1,4-diazabicyclo[2,2,2]octane, may be contained within the scope which would not hinder the object of the present invention.

In the course of reaction, a solvent can be used. The solvent may be any one as long as the reaction is not hindered. Representative examples of the solvent include, for instance, diethyl ether, diisopropyl ether, tetrahydrofuran, dioxane, dimethoxyethane, dichloromethane, chloroform, benzene, toluene, xylene, acetonitrile, acetone, methyl ethyl ketone, ethyl acetate, hydrous solvents thereof, water, and the like. Among them, from the viewpoint of industrial productivity, water can be favorably used.

When the reaction is carried out in the absence of the inorganic base, the halogenoalkylsulfonyl chloride can be reacted with the amine represented by the general formula (III), for instance, by adding the halogenoalkylsulfonyl chloride dropwise with stirring to the amine dissolved in a solvent or to the amine without a solvent, or by adding dropwise the amine with stirring under ice-cooling to the halogenoalkylsulfonyl chloride dissolved in a solvent or to the halogenoalkylsulfonyl chloride without a solvent. Alternatively, when the reaction is carried out in the presence of the inorganic base, the reaction may be carried out, for instance, by mixing the halogenoalkylsulfonyl chloride with a solvent, adding thereto an inorganic base under ice-cooling, and thereafter adding thereto the amine dropwise with stirring, or by mixing the amine with a solvent, adding thereto the base under ice-cooling, and thereafter adding thereto the halogenoalkylsulfonyl chloride dropwise with stirring.

The atmosphere in the course of reaction is not limited to specified ones, and it may be air or an inert gas such as nitrogen gas. The temperature inside the reaction system may be usually a temperature of 0°C to the boiling point of the solvent.

The conclusion of reaction can be regarded as a point at which the starting materials disappeared when observed, for instance, by thin layer chromatography.

After the conclusion of reaction, the resulting reaction mixture is washed with water, saturated brine, or the like, and dried over, for instance, anhydrous magnesium sulfate, or the like, and the dried product is concentrated under reduced pressure, to give a crude product. The halogenoalkylsulfonamide derivative represented by the general formula (Ia) can be obtained by purifying the crude product by means of, for instance, silica gel chromatography, recrystallization, or the like.

When water is used as a solvent, the reaction mixture obtained after the conclusion of reaction is extracted with, for instance, diethyl ether, chloroform, ethyl acetate, or the like, and thereafter the same procedures as above can be carried out.

According to the process of the present invention, the halogenoalkylsulfonamide derivative represented by the general formula (Ia) can be industrially and advantageously obtained.

Next, the process for preparing the halogenoalkylsulfonamide derivative represented by the general formula (Ib): 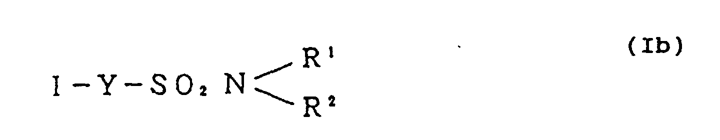 wherein R¹, R² and Y are as defined above,
will be described.

The halogenoalkylsulfonamide derivative represented by the general formula (Ib) can be obtained by reacting the halogenoalkylsulfonamide derivative represented by the general formula (Ia) with sodium iodide.

In the course of reaction, a solvent can be used. As the solvent, any ones can be used as long as the reaction is not hindered. There can be cited, for instance, methyl ethyl ketone, acetone, and the like.

The halogenoalkylsulfonamide derivative represented by the general formula (Ia) is dissolved in a solvent, and thereafter sodium iodide mentioned above is added thereto.

It is desired that the amount of sodium iodide is usually 1 to 3 mol, preferably 2 mol, per one mol of the halogenoalkylsulfonamide derivative represented by the general formula (Ia).

After sodium iodide is added and dissolved therein, the resulting solution is heated to a temperature of room temperature to a boiling point of the solvent.

The heating time is not limited to specified ones, and heating can be carried out until the conclusion of the reaction.

The conclusion of the reaction can be regarded as a point at which the starting materials disappeared when observed, for instance, by thin layer chromatography.

After the conclusion of the reaction, the resulting reaction mixture is washed with, for instance, water, saturated brine, and the like, dried over anhydrous magnesium sulfate, or the like, and thereafter the dried product is concentrated under reduced pressure, to give a crude product. The halogenoalkylsulfonamide derivative represented by the general formula (Ib) can be obtained by purifying the crude product by means of, for instance, silica gel chromatography, recrystallization, or the like.

Next, the process for preparing the halogenoalkylsulfonyl chloride represented by the general formula (IIa) will be described.

The halogenoalkylsulfonyl chloride represented by the general formula (IIa) can be obtained by reacting a sodium bromoalkylsulfonate represented by the general formula (IV):

Br-Y-SO₃Na (IV)

wherein Y is as defined above,
with a chlorinating agent.

Representative examples of the sodium bromoalkylsulfonate represented by the general formula (IV) include sodium 5-bromopentanesulfonate, sodium 6-bromohexanesulfonate, sodium 7-bromoheptanesulfonate, sodium 8-bromooctanesulfonate, sodium 2-bromohexanesulfonate, sodium 3-bromohexanesulfonate, sodium 5-bromohexanesulfonate, sodium 9-bromononanesulfonate, sodium 10-bromodecanesulfonate, sodium 11-bromoundecanesulfonate and sodium 12-bromododecanesulfonate.

In the general formula (IV), it is particularly preferable that Y is an alkylene group having 5 to 8 carbon atoms.

The sodium bromoalkylsulfonate represented by the general formula (IV) can be obtained by known methods (*Org. Synth*., Coll. Vol. II, 558; WO95/19345 Pamphlet).

As the chlorinating agent used in the course of reaction, there can be cited, for instance, phosphorus pentachloride, phosphorus trichloride, phosphorus oxychloride, chlorosulfonic acid, sulfuryl chloride, thionyl chloride, phosgene, oxalyl chloride, and the like.

When the sodium bromoalkylsulfonate represented by the general formula (IV) is reacted with the chlorinating agent, a catalyst can be used.

As the catalyst, there can be cited, for instance dimethylformamide, or the like.

In the course of reaction, the solution may be heated to room temperature to 150°C or so. The heating time is not limited to specified ones, and it may be a period until which the reaction is finished.

After the conclusion of reaction, the resulting reaction solution is washed with, for instance, water, saturated brine, and the like, dried over, for instance, calcium chloride, or the like, and thereafter the dried product is concentrated under reduced pressure, to give the halogenoalkylsulfonyl chloride represented by the general formula (IIa).

Next, the present invention will be described in further detail on the basis of the working examples, without intending to limit the scope of the present invention only to these examples.

### Example 1 [Preparation of N-Cyclopropyl-6-chlorohexanesulfonamide]

1 M-aqueous sodium hydroxide (3.0 mM) was added to a liquid mixture of 6-chlorohexanesulfonyl chloride (657 mg, 3.0 mM) synthesized referring to the literatures of *Bull. Soc. Chim. Belges*., **74**, 21 (1965) and *J. Org. Chem*., **52**, 2162 (1987) and water (9 ml) under ice-cooling. Cyclopropylamine (206 mg, 3.6 mM) was added dropwise thereto with stirring. Thereafter, the mixture was stirred at the same temperature for 1.5 hours. The mixture was diluted with diethyl ether (30 ml), and an organic layer was collected. The extract was washed with water and saturated brine, and thereafter dried over anhydrous magnesium sulfate.

Subsequently, the solvent was removed by evaporation *in vacuo*. The residue was subjected to silica gel column chromatography. N-cyclopropyl-6-chlorohexanesulfonamide (588 mg) having the following physical properties was obtained from a fraction eluted from chloroform as colorless crystals (yield 82%).
Melting Point: 38° - 39°C (diethyl ether)
IR νmax (KBr) cm⁻¹: 3267 (NH), 1317, 1135 (SO₂)
Mass Spectroscopy (C₉H₁₈ClNO₂S) EI: m/z 239 (M⁺)
EI-HRMS (C₉H₁₈ClNO₂S)
Calculated: 239.0745, Found: 239.0746
¹H-NMR (CDCl₃) δ: 0.64-0.78 (4H, m, CHCH₂×2), 1.43-1.58 (4H, m, ClCH₂CH₂CH₂CH₂), 1.72-1.89 (4H, m, ClCH₂CH₂, SCH₂CH₂), 2.54-2.61 (1H, m, CH), 3.06-3.11 (2H, m, SCH₂), 3.55 (2H, t, J = 6.5 Hz, ClCH₂), 4.79 (1H, brs, NH)
Elemental Analysis (C₉H₁₈ClNO₂S)
Calculated: C, 45.08; H, 7.57; N, 5.84, Found: C, 44.82; H, 7.50; N, 5.88

### Example 2 [Preparation of N-Cyclopropyl- 6-chlorohexanesulfonamide]

The reaction was carried out in the same manner as in Example 1 except for using diethyl ether in place of water in Example 1, to give N-cyclopropyl-6-chlorohexanesulfonamide (674 mg) (yield 94%).

### Example 3 [Preparation of N-Methyl- 6-chlorohexanesulfonamide]

6-Chlorohexanesulfonyl chloride (3.29 g, 15 mM) was added dropwise to a solution of diethyl ether (30 ml) and a 40% aqueous solution of methylamine (2.56 g, 33 mM) with stirring under ice-cooling. Thereafter, the mixture was stirred at the same temperature for 20 minutes. The reaction mixture was washed twice with water (10 ml) and then with saturated brine (10 ml), and thereafter dried over anhydrous magnesium sulfate.

Subsequently, the solvent was removed by evaporation *in vacuo*. The crude product was subjected to silica gel column chromatography. N-Methyl-6-chlorohexanesulfonamide (3.04 g) having the following physical properties was obtained from a fraction eluted from a mixed solution of ethyl acetate-n-hexane (1:4)-(1:3) as a colorless oily product (yield 95%).
IR νmax (neat) cm⁻¹: 3302 (NH), 1320, 1148 (SO₂)
Mass Spectroscopy (C₇H₁₆ClNO₂S) EI: m/z 214 (M⁺ +1), CI: m/z 214 (M⁺ +1)
EI-HRMS (C₇H₁₆ClNO₂S+H⁺)
Calculated: 214.0668, Found: 214.0670
¹H-NMR (CDCl₃) δ: 1.42-1.57 (4H, m, ClCH₂CH₂CH₂CH₂), 1.75-1.89 (4H, m, ClCH₂CH₂, SCH₂CH₂), 2.81 (3H, d, J = 5.3 Hz, NCH₃), 3.00-3.05 (2H, m, SCH₂), 3.55 (2H, t, J = 6.6 Hz, ClCH₂), 4.31 (1H, brq, J = 5.3 Hz, NH)

### Example 4 [Preparation of N,N-Dimethyl- 6-chlorohexanesulfonamide]

6-Chlorohexanesulfonyl chloride (6.57 g, 30 mM) was added dropwise to a solution of diethyl ether (60 ml) and a 12% acetonitrile solution of dimethylamine (24.8 g, 66 mM) with stirring under ice-cooling. Thereafter, the mixture was stirred at the same temperature for 20 minutes. The reaction mixture was concentrated *in vacuo*. Thereafter, the concentrate was diluted with diethyl ether (60 ml). The resulting solution was washed twice with water (10 ml) and then with saturated brine (10 ml), and thereafter dried over anhydrous magnesium sulfate. Subsequently, the solvent was removed by evaporation *in vacuo*. The crude product was subjected to silica gel column chromatography. N,N-dimethyl-6-chlorohexanesulfonamide (6.59 g) having the following physical properties was obtained from a fraction eluted from a mixed solution of ethyl acetate-n-hexane (1:4) as a pale yellow oily product (yield 97%).
IR νmax (neat) cm⁻¹: 1335, 1143 (SO₂)
Mass Spectroscopy (C₈H₁₈ClNO₂S) EI: m/z 228 (M⁺ +1), CI: m/z 228 (M⁺ +1)
EI-HRMS (C₈H₁₈ClNO₂S+H⁺)
Calculated; 228.0824, Found: 228.0824
¹H-NMR (CDCl₃) δ: 1.41-1.60 (4H, m, ClCH₂CH₂CH₂CH₂), 1.71-1.88 (4H, m, ClCH₂CH₂, SCH₂CH₂), 2.87 (6H, s, CH₃×2), 2.89-2.94 (2H, m, SCH₂), 3.54 (2H, t, J = 6.6 Hz, ClCH₂)

### Example 5 [Preparation of N-Ethyl- 6-chlorohexanesulfonamide]

The reaction was carried out in the same manner as in Example 3 except for using a 70% aqueous solution of ethylamine (2.41 g, 33 mM) in place of methylamine in Example 3, to give N-ethyl-6-chlorohexanesulfonamide (3.33 g) having the following physical properties as a colorless oily product (yield 98%).
IR νmax (neat) cm⁻¹: 3289 (NH), 1320, 1147 (SO₂)
Mass Spectroscopy (C₈H₁₈ClNO₂S) EI: m/z 228 (M⁺ +1), CI: m/z 228 (M⁺ +1)
EI-HRMS (C₈H₁₈ClNO₂S+H⁺)
Calculated: 228.0824, Found: 228.0827
¹H-NMR (CDCl₃) δ: 1.23 (3H, t, J = 7.2 Hz, CH₃), 1.42-1.58 (4H, m, ClCH₂CH₂CH₂CH₂), 1.72-1.89 (4H, m, ClCH₂CH₂, SCH₂CH₂), 3.00-3.05 (2H, m, SCH₂), 3.18 (2H, quint, J = 6.8 Hz, NCH₂), 3.55 (2H, t, J = 6.5 Hz, ClCH₂), 4.31 (1H, brt, J = 6.8 Hz, NH)

### Example 6 [Preparation of N,N-Diethyl- 6-chlorohexanesulfonamide]

The reaction was carried out in the same manner as in Example 3 except for using diethylamine (2.41 g, 33 mM) in place of methylamine in Example 3, to give N,N-diethyl-6-chlorohexanesulfonamide (3.56 g) having the following physical properties as a pale yellow oily product (yield 93%).
IR νmax (neat) cm⁻¹: 1329, 1140 (SO₂)
Mass Spectroscopy (C₁₀H₂₂ClNO₂S) EI: m/z 255 (M⁺)
EI-HRMS (C₁₀H₂₂ClNO₂S)
Calculated: 255.1059, Found: 255.1064
¹H-NMR (CDCl₃) δ: 1.21 (6H, t, J = 7.1 Hz, CH₃×2), 1.39-1.55 (4H, m, ClCH₂CH₂CH₂CH₂), 1.73-1.87 (4H, m, ClCH₂CH₂, SCH₂CH₂), 2.89-2.95 (2H, m, SCH₂), 3.30 (4H, q, J = 7.1 Hz, CH₃CH₂×2), 3.54 (2H, t, J = 6.5 Hz, ClCH₂),

### Example 7 [Preparation of N-n-Propyl- 6-chlorohexanesulfonamide]

The reaction was carried out in the same manner as in Example 3 except for using n-propylamine (1.95 g, 33 mM) in place of methylamine in Example 3, to give N-n-propyl-6-chlorohexanesulfonamide (3.51 g) having the following physical properties as a pale yellow oily product (yield 97%).
IR νmax (neat) cm⁻¹: 3291 (NH), 1320, 1144 (SO₂)
Mass Spectroscopy (C₉H₂₀ClNO₂S) EI: m/z 242 (M⁺ +1), CI: m/z 242 (M⁺ +1)
EI-HRMS (C₉H₂₀ClNO₂S+H⁺)
Calculated: 242.0981, Found: 242.0982
¹H-NMR (CDCl₃) δ: 0.97 (3H, t, J = 7.4 Hz, CH₃), 1.49 (4H, m, ClCH₂CH₂CH₂CH₂), 1.60 (2H, m, CH₃CH₂), 1.75-1.92 (4H, m, ClCH₂CH₂, SCH₂CH₂), 2.99-3.04 (2H, m, SCH₂), 3.08 (2H, q, J = 7.4 Hz, NCH₂), 3.55 (2H, t, J = 6.6 Hz, ClCH₂), 4.38 (1H, brt, J = 7.4 Hz, NH)

### Example 8 [Preparation of N-Isopropyl-6-chlorohexanesulfonamide]

The reaction was carried out in the same manner as in Example 3 except for using isopropylamine (1.95 g, 33 mM) in place of methylamine in Example 3, to give N-isopropyl-6-chlorohexanesulfonamide (3.53 g) having the following physical properties as a pale yellow oily product (yield 97%).
IR νmax (neat) cm⁻¹: 3286 (NH), 1319, 1133 (SO₂)
Mass Spectroscopy (C₉H₂₀ClNO₂S) EI: m/z 242 (M⁺ +1), CI: m/z 242 (M⁺ +1)
EI-HRMS (C₉H₂₀ClNO₂S+H⁺)
Calculated: 242.0980, Found: 242.0976
¹H-NMR (CDCl₃) δ: 1.25 (6H, d, J = 6.6 Hz, CH₃×2), 1.44-1.53 (4H, m, ClCH₂CH₂CH₂CH₂), 1.75-1.89 (4H, m, ClCH₂CH₂, SCH₂CH₂), 2.99-3.03 (2H, m, SCH₂), 3.54 (2H, t, J = 6.6 Hz, ClCH₂), 3.65 (1H, m, CH), 4.22 (1H, brd, J = 9.0 Hz, NH)

### Example 9 [Preparation of N-n-Butyl- 6-chlorohexanesulfonamide]

The reaction was carried out in the same manner as in Example 3 except for using n-butylamine (2.41 g, 33 mM) in place of methylamine in Example 3, to give N-n-butyl-6-chlorohexanesulfonamide (3.79 g) having the following physical properties as a pale yellow oily product (yield 99%).
IR νmax (neat) cm⁻¹: 3286 (NH), 1320, 1143 (SO₂)
Mass Spectroscopy (C₁₀H₂₂ClNO₂S) EI: m/z 256 (M⁺ +1), CI: m/z 256 (M⁺ +1)
EI-HRMS (C₁₀H₂₂ClNO₂S+H⁺)
Calculated: 256.1137, Found: 256.1130
¹H-NMR (CDCl₃) δ: 0.95 (3H, t, J = 7.2 Hz, CH₃), 1.38 (2H, m, CH₃CH₂), 1.49 (4H, m, ClCH₂CH₂CH₂CH₂), 1.53 (2H, m, NCH₂CH₂), 1.72-1.88 (4H, m, ClCH₂CH₂, SCH₂CH₂), 2.99-3.04 (2H, m, SCH₂), 3.11 (2H, q, J = 6.7 Hz, NCH₂), 3.55 (2H, t, J = 6.5 Hz, ClCH₂), 4.37 (1H, brt, J = 6.7 Hz, NH)

### Example 10 [Preparation of N-tert-Butyl- 6-chlorohexanesulfonamide]

The reaction was carried out in the same manner as in Example 3 except for using tert-butylamine (2.41 g, 33 mM) in place of methylamine in Example 3, to give N-tert-butyl-6-chlorohexanesulfonamide (3.62 g) as a pale yellow oily product (yield 94%).
IR νmax (neat) cm⁻¹: 3282 (NH), 1317, 1139 (SO₂)
Mass Spectroscopy (C₁₀H₂₂ClNO₂S) EI: m/z 256 (M⁺ +1), CI: m/z 256 (M⁺ +1)
EI-HRMS (C₁₀H₂₂ClNO₂S+H⁺)
Calculated: 256.1137, Found: 256.1138
¹H-NMR (CDCl₃) δ: 1.38 (9H, s, CH₃×3), 1.44-1.53 (4H, m, ClCH₂CH₂CH₂CH₂), 1.76-1.89 (4H, m, ClCH₂CH₂, SCH₂CH₂), 3.02-3.07 (2H, m, SCH₂), 3.54 (2H, t, J = 6.6 Hz, ClCH₂), 4.28 (1H, brs, NH)

### Example 11 [Preparation of N-n-Pentyl- 6-chlorohexanesulfonamide]

6-Chlorohexanesulfonyl chloride (3.29 g, 15 mM) was added dropwise to a solution of diethyl ether (30 ml) and n-pentylamine (2.88 g, 33 mM) with stirring under ice-cooling. Thereafter, the mixture was stirred at the same temperature for 20 minutes. The reaction mixture was washed twice with water (10 ml) and then with saturated brine (10 ml), and thereafter dried over anhydrous magnesium sulfate. Subsequently, the solvent was removed by evaporation *in vacuo*, to give crude N-n-pentyl-6-chlorohexanesulfonamide (4.10 g) as pale yellow crystals (yield 100%).

Subsequently, the resulting crude N-n-pentyl-6-chlorohexanesulfonamide was recrystallized from n-hexane. N-n-Pentyl-6-chlorohexanesulfonamide (3.66 g) having the following physical properties was obtained as colorless crystals (yield 90%).
Melting Point: 47° - 48°C (n-hexane)
IR νmax (KBr) cm⁻¹: 3283 (NH), 1314, 1135 (SO₂)
Mass Spectroscopy (C₁₁H₂₄ClNO₂S) EI: m/z 270 (M⁺ +1), CI: m/z 270 (M⁺ +1)
EI-HRMS (C₁₁H₂₄ClNO₂S+H⁺)
Calculated: 270.1293, Found: 270.1293
¹H-NMR (CDCl₃) δ: 0.92 (3H, t, J = 6.7 Hz, CH₃), 1.27-1.41 (4H, m, CH₃CH₂CH₂), 1.49 (4H, m, ClCH₂CH₂CH₂CH₂), 1.57 (2H, m, NCH₂CH₂), 1.72-1.89 (4H, m, ClCH₂CH₂, SCH₂CH₂), 3.00-3.04 (2H, m, SCH₂), 3.11 (2H, q, J = 6.6 Hz, NCH₂), 3.55 (2H, t, J = 6.6 Hz, ClCH₂), 4.32 (1H, brt, J = 6.6 Hz, NH)
Elemental Analysis (C₁₁H₂₄ClNO₂S)
Calculated: C, 48.96; H, 8.97; N, 5.19, Found: C, 48.86; H, 9.21; N, 5.35

### Example 12 [Preparation of N-n-Hexyl- 6-chlorohexanesulfonamide]

The reaction was carried out in the same manner as in Example 11 except for using n-hexylamine (3.34 g, 33 mM) in place of n-pentylamine in Example 11, to give crude N-n-hexyl-6-chlorohexanesulfonamide (4.30 g) as pale yellow crystals (yield 100%). The resulting crude product was recrystallized from n-hexane. N-n-Hexyl-6-chlorohexanesulfonamide (4.12 g) having the following physical properties was obtained as colorless crystals (yield 97%).
Melting Point: 51° - 52°C (n-hexane)
IR νmax (KBr) cm⁻¹: 3294 (NH), 1317, 1136 (SO₂)
Mass Spectroscopy (C₁₂H₂₆ClNO₂S) EI: m/z 284 (M⁺ +1), CI: m/z 284 (M⁺ +1)
EI-HRMS (C₁₂H₂₆ClNO₂S+H⁺)
Calculated: 284.1449, Found: 284.1447
¹H-NMR (CDCl₃) δ: 0.90 (3H, t, J = 6.8 Hz, CH₃), 1.32 (6H, m, CH₃CH₂CH₂CH₂), 1.49 (4H, m, ClCH₂CH₂CH₂CH₂), 1.56 (2H, m, NCH₂CH₂), 1.72-1.88 (4H, m, ClCH₂CH₂, SCH₂CH₂), 2.99-3.04 (2H, m, SCH₂), 3.11 (2H, q, J = 6.8 Hz, NCH₂), 3.55 (2H, t, J = 6.6 Hz, ClCH₂), 4.33 (1H, brt, J = 6.8 Hz, NH)
Elemental Analysis (C₁₂H₂₆ClNO₂S)
Calculated: C, 50.77; H, 9.23; N, 4.94, Found: C, 50.52; H, 9.34; N, 5.04

### Example 13 [Preparation of N-Cyclobutyl-6-chlorohexanesulfonamide]

The reaction was carried out in the same manner as in Example 11 except for using cyclobutylamine (2.35 g, 33 mM) in place of n-pentylamine in Example 11, to give crude N-cyclobutyl-6-chlorohexanesulfonamide (3.88 g) as pale yellow crystals (yield 100%).

The resulting pale yellow crystals of N-cyclobutyl-6-chlorohexanesulfonamide were recrystallized from diethyl ether. Colorless crystals were obtained. Thereafter, the physical properties thereof were evaluated. The results are as follows.
Melting Point: 57° - 58°C (diethyl ether)
IR νmax (KBr) cm⁻¹: 3281 (NH), 1319, 1138 (SO₂)
Mass Spectroscopy (C₁₀H₂₀ClNO₂S) EI: m/z 254 (M⁺ +1), CI: m/z 254 (M⁺ +1)
EI-HRMS (C₁₀H₂₀ClNO₂S+H⁺)
Calculated: 254.0981, Found: 254.0981
¹H-NMR (CDCl₃) δ: 1.41-1.56 (4H, m, ClCH₂CH₂CH₂CH₂), 1.58-2.04 (8H, m, ClCH₂CH₂, SCH₂CH₂, CHCH₂CH₂, CHCHH×2), 2.31-2.44 (2H, m, CHCHH×2), 2.95-3.00 (2H, m, SCH₂), 3.55 (2H, t, J = 6.6 Hz, ClCH₂), 3.91 (1H, m, CH), 4.64 (1H, brd, J = 8.9 Hz, NH)
Elemental Analysis (C₁₀H₂₀ClNO₂S)
Calculated: C, 47.32; H, 7.94; N, 5.52, Found: C, 47.21; H, 8.09; N, 5.75

### Example 14 [Preparation of N-Cyclopentyl- 6-chlorohexanesulfonamide]

The reaction was carried out in the same manner as in Example 11 except for using cyclopentylamine (2.81 g, 33 mM) in place of n-pentylamine in Example 11, to give crude N-cyclopentyl-6-chlorohexanesulfonamide (4.00 g) as pale brown crystals (yield 100%).

The resulting pale brown crystals of N-cyclopentyl-6-chlorohexanesulfonamide were recrystallized from diethyl ether. Thereafter, the physical properties thereof were evaluated. The results are as follows.
Melting Point: 61° - 62°C (diethyl ether)
IR νmax (KBr) cm⁻¹: 3276 (NH), 1317, 1130 (SO₂)
Mass Spectroscopy (C₁₁H₂₂ClNO₂S) EI: m/z 267 (M⁺) EI-HRMS (C₁₁H₂₂ClNO₂S)
Calculated: 267.1058, Found: 267.1056
¹H-NMR (CDCl₃) δ: 1.42-1.89 (14H, m, CH₂×6, CHCHH×2), 1.95-2.07 (2H, m, CHCHH×2), 3.00-3.05 (2H, m, SCH₂), 3.55 (2H, t, J = 6.6 Hz, ClCH₂), 3.71-3.82 (1H, m, CH), 4.34 (1H, brd, J = 7.4 Hz, NH)
Elemental Analysis (C₁₁H₂₂ClNO₂S)
Calculated: C, 49.33; H, 8.28; N, 5.23, Found: C, 49.34; H, 8.44; N, 5.30

### Example 15 [Preparation of N-Cyclohexyl- 6-chlorohexanesulfonamide]

The reaction was carried out in the same manner as in Example 11 except for using cyclohexylamine (3.27 g, 33 mM) in place of n-pentylamine in Example 11, to give crude N-cyclohexyl-6-chlorohexanesulfonamide (4.30 g) as pale yellow crystals (yield 100%).

The resulting pale yellow crystals of N-cyclohexyl-6-chlorohexanesulfonamide were recrystallized from diethyl ether. Thereafter, the physical properties thereof were evaluated. The results are as follows.
Melting Point: 62° - 63°C (diethyl ether)
IR νmax (KBr) cm⁻¹: 3277 (NH), 1317, 1130 (SO₂)
Mass Spectroscopy (C₁₂H₂₄ClNO₂S) EI: m/z 281 (M⁺)
EI-HRMS (C₁₂H₂₄ClNO₂S)
Calculated: 281.1215, Found: 281.1216
¹H-NMR (CDCl₃) δ: 1.11-1.67 (10H, m, CH₂×5), 1.71-1.89 (6H, m, CH₂×2, CHCHH×2), 1.95-2.01 (2H, m, CHCHH×2), 2.99-3.04 (2H, m, SCH₂), 3.22-3.35 (1H, m, CH), 3.55 (2H, t, J = 6.5 Hz, ClCH₂), 4.24 (1H, brd, J = 7.7 Hz, NH)
Elemental Analysis (C₁₂H₂₄ClNO₂S)
Calculated: C, 51.14; H, 8.58; N, 4.97, Found: C, 51.06; H, 8.79; N, 5.16

### Example 16 [Preparation of 5-Bromopentanesulfonyl Chloride]

Thionyl chloride (11.7 ml, 160 mM) and N,N-dimethylformamide (one drop) were added to sodium 5-bromopentanesulfonate (5.06 g, 20 mM) prepared by a process described in *Org. Synth*., Coll. Vol. II, 558 or WO95/19345 Pamphlet. The mixture was refluxed for 4 hours. The reaction mixture was concentrated *in vacuo*. The concentrate was diluted with benzene (50 ml). The resulting solution was washed twice with water (15 ml) and then with saturated brine (10 ml), and thereafter dried over calcium chloride. The solvent was removed by evaporation *in vacuo*. Crude 5-bromopentanesulfonyl chloride (2.13 g) having the following physical properties was obtained as a yellow oily product (yield 43%).
IR νmax (neat) cm⁻¹: 1368, 1163 (SO₂)
Mass Spectroscopy (C₅H₁₀BrClO₂S) EI: m/z 249 (M⁺ +1), CI: m/z 249 (M⁺ +1)
EI-HRMS (C₅H₁₀BrClO₂S+H⁺)
Calculated: 248.9351, Found: 248.9342
¹H-NMR (CDCl₃) δ: 1.65-1.75 (2H, m, CH₂), 1.97 (2H, m, BrCH₂CH₂), 2.11 (2H, m, SCH₂CH₂), 3.45 (2H, t, J = 6.5 Hz, BrCH₂), 3.71 (2H, t, J = 7.8 Hz, SCH₂)

### Example 17 [Preparation of N-Cyclopropyl- 5-bromopentanesulfonamide]

The reaction was carried out in the same manner as in Example 3 except for using of 5-bromopentanesulfonyl chloride (2.09 g, 8.37 mM) obtained in Example 16, cyclopropylamine (1.00 g, 17.59 mM), and diethyl ether (20 ml) in Example 3, to give N-cyclopropyl-5-bromopentanesulfonamide (1.19 g) having the following physical properties as a colorless waxy product (yield 53%).
IR νmax (neat) cm⁻¹: 3271 (NH), 1316, 1136 (SO₂)
Mass Spectroscopy (C₈H₁₆BrNO₂S) EI: m/z 269 (M⁺)
EI-HRMS (C₈H₁₆BrNO₂S)
Calculated: 269.0084, Found: 269.0089
¹H-NMR (CDCl₃) δ: 0.64-0.77 (4H, m, CHCH₂×2), 1.56-1.66 (2H, m, BrCH₂CH₂CH₂), 1.79-1.96 (4H, m, BrCH₂CH₂, SCH₂CH₂), 2.54-2.61 (1H, m, CH), 3.09 (2H, t, J = 7.9 Hz, SCH₂), 3.42 (2H, t, J = 6.6 Hz, BrCH₂), 4.88 (1H, brs, NH)

### Example 18 [Preparation of 6-Bromohexanesulfonyl Chloride]

The reaction was carried out in the same manner as in Example 16 except for using sodium 6-bromohexanesulfonate (5.00 g, 18.7 mM) prepared by a process described in *Org*. *Synth*., Coll. Vol. II, 558 or WO95/19345 Pamphlet. Thionyl chloride (10.3 ml, 140.4 mM) in Example 16, to give crude 6-bromohexanesulfonyl chloride (1.61 g) having the following physical properties as a pale yellow oily product (yield 33%).
IR νmax (neat) cm⁻¹: 1360, 1160 (SO₂)
Mass Spectroscopy (C₆H₁₂BrClO₂S) EI: m/z 263 (M⁺ +1), CI: m/z 263 (M⁺ +1)
EI-HRMS (C₆H₁₂BrClO₂S+H⁺)
Calculated: 262.9508, Found: 262.9509
¹H-NMR (CDCl₃) δ: 1.56 (4H, m, CH₂×2), 1.92 (2H, m, BrCH₂CH₂), 2.09 (2H, m, SCH₂CH₂), 3.43 (2H, t, J = 6.6 Hz, BrCH₂), 3.69 (2H, t, J = 7.8 Hz, SCH₂)

### Example 19 [Preparation of N-Cyclopropyl- 6-bromohexanesulfonamide]

The reaction was carried out in the same manner as in Example 3 except for using 6-bromohexanesulfonyl chloride (1.78 g, 6.78 mM) obtained in Example 18 in place of 6-chlorohexanesulfonyl chloride in Example 3, cyclopropylamine (774 mg, 13.56 mM) and diethyl ether (10 ml), to give N-cyclopropyl-6-bromohexanesulfonamide (1.52 g) having the following physical properties as a colorless waxy product (yield 79%).
IR νmax (neat) cm⁻¹: 3250 (NH), 1310, 1140 (SO₂)
Mass Spectroscopy (C₉H₁₈BrNO₂S) EI: m/z 283 (M⁺)
EI-HRMS (C₉H₁₈BrNO₂S)
Calculated: 283.0240, Found: 283.0246
¹H-NMR (CDCl₃) δ: 0.65-0.79 (4H, m, CHCH₂×2), 1.51 (4H, m, BrCH₂CH₂CH₂CH₂), 1.75-1.94 (4H, m, BrCH₂CH₂, SCH₂CH₂), 2.56-2.62 (1H, m, CH), 3.07-3.12 (2H, m, SCH₂), 3.43 (2H, t, J = 6.7 Hz, BrCH₂), 4.66 (1H, brs, NH)

### Example 20 [Preparation of 7-Bromoheptanesulfonyl Chloride]

The reaction was carried out in the same manner as in Example 16 except for using sodium 7-bromoheptanesulfonate (5.62 g, 20 mM) prepared by a process described in *Org*. *Synth*., Coll. Vol. II, 558 or WO95/19345 Pamphlet, and thionyl chloride (11.7 ml, 160 mM) in Example 16, to give crude 7-bromoheptanesulfonyl chloride (2.56 g) having the following physical properties as a pale yellow oily product (yield 46%).
IR νmax (neat) cm⁻¹: 1374, 1166 (SO₂)
Mass Spectroscopy (C₇H₁₄BrClO₂S) EI: m/z 277 (M⁺ +1), CI: m/z 277 (M⁺ +1)
EI-HRMS (C₇H₁₄BrClO₂S+H⁺)
Calculated: 276.9664, Found: 276.9667
¹H-NMR (CDCl₃) δ: 1.36-1.59 (6H, m, CH₂×3), 1.89 (2H, m, BrCH₂CH₂), 2.08 (2H, m, SCH₂CH₂), 3.43 (2H, t, J = 6.7 Hz, BrCH₂), 3.68 (2H, t, J = 7.8 Hz, SCH₂)

### Example 21 [Preparation of N-Cyclopropyl- 7-bromoheptanesulfonamide]

The reaction was carried out in the same manner as in Example 3 except for using 7-bromoheptanesulfonyl chloride (2.51 g, 9.04 mM) obtained in Example 20 in place of 6-chlorohexanesulfonyl chloride in Example 3, cyclopropylamine (1.08 g, 18.99 mM) and diethyl ether (20 ml), to give N-cyclopropyl-7-bromoheptanesulfonamide (1.20 g) having the following physical properties as a colorless waxy product (yield 44%).
IR νmax (neat) cm⁻¹: 3272 (NH), 1302, 1139 (SO₂)
Mass Spectroscopy (C₁₀H₂₀BrNO₂S) EI: m/z 297 (M⁺)
EI-HRMS (C₁₀H₂₀BrNO₂S)
Calculated: 297.0397, Found: 297.0394
¹H-NMR (CDCl₃) δ: 0.63-0.78 (4H, m, CHCH₂×2), 1.31-1.52 (6H, m, CH₂×3), 1.74-1.91 (4H, m, BrCH₂CH₂, SCH₂CH₂), 2.54-2.61 (1H, m, CH), 3.05-3.10 (2H, m, SCH₂), 3.41 (2H, t, J = 6.7 Hz, BrCH₂), 4.80 (1H, brs, NH)

### Example 22 [Preparation of 8-Bromooctanesulfonyl Chloride]

Thionyl chloride (11.7 ml, 160 mM) and N,N-dimethylformamide (one drop) were added to sodium 8-bromooctanesulfonate (5.90 g, 20 mM) prepared by a process described in *Org. Synth*., Coll. Vol. II, 558 or WO95/19345 Pamphlet. The mixture was refluxed for 4 hours. The reaction mixture was concentrated *in vacuo*. The concentrate was diluted with benzene (60 ml). The resulting solution was washed twice with water (15 ml) and then with saturated brine (10 ml), and thereafter dried over calcium chloride. The solvent was removed by evaporation *in vacuo*. The crude product was subjected to silica gel column chromatography. 8-Bromooctanesulfonyl chloride (1.30 g) having the following physical properties was obtained from a fraction eluted from a mixed solution of n-hexane-benzene (2:1) as a colorless oily product (yield 22%).
IR νmax (neat) cm⁻¹: 1374, 1165 (SO₂)
Mass Spectroscopy (C₈H₁₆BrClO₂S) EI: m/z 291 (M⁺ +1), CI: m/z 291 (M⁺ +1)
EI-HRMS (C₈H₁₆BrClO₂S+H⁺)
Calculated: 290.9820, Found: 290.9805
¹H-NMR (CDCl₃) δ: 1.27-1.58 (8H, m, CH₂×4), 1.75-1.94 (2H, m, BrCH₂CH₂), 2.02-2.12 (2H, m, SCH₂CH₂), 3.43 (2H, t, J = 6.7 Hz, BrCH₂), 3.66-3.71 (2H, m, SCH₂)

### Example 23 [Preparation of N-Cyclopropyl- 8-bromooctanesulfonamide]

The reaction was carried out in the same manner as in Example 3 except for using 8-bromooctanesulfonyl chloride (1.26 g, 4.32 mM) obtained in Example 22 in place of 6-chlorohexanesulfonyl chloride in Example 3, cyclopropylamine (0.52 g, 9.05 mM) and diethyl ether (20 ml), to give N-cyclopropyl-8-bromooctanesulfonamide (1.29 g) having the following physical properties as a pale yellow waxy product (yield 96%).
IR νmax (neat) cm⁻¹: 3273 (NH), 1317, 1135 (SO₂)
Mass Spectroscopy (C₁₁H₂₂BrNO₂S) EI: m/z 311 (M⁺)
EI-HRMS (C₁₁H₂₂BrNO₂S)
Calculated: 311.0554, Found: 311.0555
¹H-NMR (CDCl₃) δ: 0.64-0.78 (4H, m, CHCH₂×2), 1.27-1.53 (8H, m, CH₂×4), 1.73-1.91 (4H, m, BrCH₂CH₂, SCH₂CH₂), 2.54-2.62 (1H, m, CH), 3.05-3.10 (2H, m, SCH₂), 3.41 (2H, t, J = 6.8 Hz, BrCH₂), 4.75 (1H, brs, NH)

### Example 24 [Preparation of N-Cyclopropyl- 6-iodohexanesulfonamide]

Sodium iodide (750 mg, 5.00 mM) was added to a solution of methyl ethyl ketone (8 ml) and N-cyclopropyl-6-chlorohexanesulfonamide (600 mg, 2.50 mM) obtained in Example 1. The mixture was refluxed under heating for 4 hours. The reaction mixture was concentrated *in vacuo*. The concentrate was diluted with ethyl acetate (30 ml). The resulting solution was washed twice with water (10 ml) end then with saturated brine (10 ml), and thereafter dried over anhydrous magnesium sulfate.

Subsequently, the solvent was removed by evaporation *in vacuo*. The crude product was subjected to silica gel column chromatography. N-Cyclopropyl-6-iodohexanesulfonamide (740 mg) having the following physical properties was obtained from a fraction eluted from a mixed solution of ethyl acetate-n-hexane (1:1) as a pale yellow waxy product (yield 89%).
IR νmax (neat) cm⁻¹: 3275 (NH), 1313, 1143 (SO₂)
Mass Spectroscopy (C₉H₁₈INO₂S) EI: m/z 332 (M⁺ +1), CI: m/z 332 (M⁺ +1)
EI-HRMS (C₉H₁₈INO₂S+H⁺)
Calculated: 332.0180, Found: 332.0176
¹H-NMR (CDCl₃) δ: 0.65-0.78 (4H, m, CHCH₂×2), 1.41-1.55 (4H, m, ICH₂CH₂CH₂CH₂), 1.72-1.95 (4H, m, ICH₂CH₂, SCH₂CH₂), 2.55-2.62 (1H, m, CH), 3.06-3.11 (2H, m, SCH₂), 3.20 (2H, t, J = 6.9 Hz, ICH₂), 4.72 (1H, brs, NH)

### Example 25 [Preparation of N-(2-Hydroxyethyl)-6-chlorohexanesulfonamide]

A diethyl ether solution (15 ml) of 6-chlorohexanesulfonyl chloride (3.29 g, 15 mM) was added dropwise to a suspension of diethyl ether (15 ml) and ethanolamine (2.02 g, 33 mM) under ice-cooling. Thereafter, the mixture was stirred at room temperature for 1 hour. Water was added to the reaction mixture. The resulting solution was extracted with ethyl acetate (50 ml). The extract was dried over anhydrous magnesium sulfate.

Subsequently, the solvent was removed by evaporation *in vacuo*. The crude product was subjected to silica gel column chromatography and eluted from a mixed solution of chloroform-methanol (20:1). A by-product [N,O-bis-(6-chlorohexanesulfonyl)-ethanolamine] was obtained from a first eluted fraction in the amount of 0.35 g (yield 10.9%). A desired compound was obtained from a second eluted fraction as a colorless oily product in the amount of 3.05 g (yield 83.4%). The desired compound obtained had the following physical properties.
IR νmax (neat) cm⁻¹: 3492 (OH), 3293 (NH), 1318, 1143 (SO₂)
Mass Spectroscopy (C₈H₁₈ClNO₃S) EI: m/z 244 (M⁺ +1), CI: m/z 244 (M⁺ +1)
EI-HRMS (C₈H₁₈ClNO₃S+H⁺)
Calculated: 244.0773, Found: 244.0781
¹H-NMR (CDCl₃) δ: 1.42-1.61 (4H, m, ClCH₂CH₂CH₂CH₂), 1.81 (4H, m, ClCH₂CH₂, SCH₂CH₂), 2.38 (1H, t, J = 5.1 Hz, OH), 3.05-3.10 (2H, m, SCH₂), 3.28 (2H, q, J = 5.2 Hz, NCH₂), 3.55 (2H, t, J = 6.5 Hz, ClCH₂), 3.78 (2H, q, J = 5.1 Hz, OCH₂), 4.96 (1H, t, J = 5.2 Hz, NH)

### Example 26 [Preparation of N-(3-Hydroxypropyl)-6-chlorohexanesulfonamide]

The reaction was carried out in the same manner as in Example 25 except for using n-propanolamine (2.48 g, 33 mM) in place of ethanolamine in Example 25, to give a by-product [N,O-bis-(6-chlorohexanesulfonyl)propanolamine] (0.85 g) from a first eluted fraction (yield 25.8%), and a desired compound (2.86 g) from a second eluted fraction as a colorless waxy product (yield 73.9%). The physical properties of the desired compound are as follows.
IR νmax (neat) cm⁻¹: 3447 (OH), 3244 (NH), 1330, 1136 (SO₂)
Mass Spectroscopy (C₉H₂₀ClNO₃S) EI: m/z 258 (M⁺ +1), CI: m/z 258 (M⁺ +1)
EI-HRMS (C₉H₂₀ClNO₃S+H⁺)
Calculated: 258.0929, Found: 258.0936
¹H-NMR (CDCl₃) δ: 1.44-1.56 (4H, m, ClCH₂CH₂CH₂CH₂), 1.74-1.89 (6H, m, ClCH₂CH₂, SCH₂CH₂, NHCH₂CH₂), 1.98 (1H, t, J = 5.1 Hz, OH), 3.02-3.07 (2H, m, SCH₂), 3.30 (2H, q, J = 6.2 Hz, NCH₂), 3.56 (2H, t, J = 6.5 Hz, ClCH₂), 3.82 (2H, q, J = 5.1 Hz, OCH₂), 4.79 (1H, t, J = 6.2 Hz, NH)

### Example 27 [Preparation of N-Cyclopropyl-5-chloropentanesulfonamide]

A diethyl ether solution (15 ml) of 5-chloropentanesulfonyl chloride (3.08 g, 15 mM) prepared referring to the literature of *Bull. Soc. Chim. Belges*., 74, 21 (1965) or *J. Org. Chem*., **52**, 2162 (1987) was added dropwise to a solution of diethyl ether (15 ml) and cycloprolylamine (1.92 g, 33 mM) with stirring under ice-cooling. Thereafter, the mixture was stirred at the same temperature for 30 minutes. The reaction mixture was washed twice with water (10 ml) and then with saturated brine (10 ml), and thereafter dried over anhydrous magnesium sulfate. Subsequently, the solvent was removed by evaporation *in vacuo*. The crude product was subjected to silica gel column chromatography. N-Cyclopropyl-5-chloropentanesulfonamide (3.31 g) having the following physical properties was obtained from a fraction eluted from chloroform as colorless crystals (yield 97.6%).
Melting Point: 36° - 37°C (ethyl acetate-ligroin)
IR νmax (KBr) cm⁻¹: 3268 (NH), 1319, 1136 (SO₂)
Mass Spectroscopy (C₈H₁₆ClNO₂S) EI: m/z 225 (M⁺)
EI-HRMS (C₈H₁₆ClNO₂S)
Calculated: 225.0589, Found: 225.0587
¹H-NMR (CDCl₃) δ: 0.65-0.79 (4H, m, CHCH₂×2), 1.53-1.69 (2H, m, ClCH₂CH₂CH₂), 1.79-1.91 (4H, m, ClCH₂CH₂, SCH₂CH₂), 2.56-2.63 (1H, m, CH), 3.11 (2H, t, J = 7.9 Hz, SCH₂), 3.57 (2H, t, J = 6.4 Hz, ClCH₂), 4.75 (1H, s, NH)
Elemental Analysis (C₈H₁₆ClNO₂S)
Calculated: C, 42.56; H, 7.14; N, 6.21, Found: C, 42.42; H, 6.92; N, 6.09

### Example 28 [Preparation of N-Isopropyl- 5-chloropentanesulfonamide]

The reaction was carried out in the same manner as in Example 27 except for using isopropylamine (1.95 g, 33 mM) in place of cyclopropylamine in Example 27, to give N-isopropyl-5-chloropentanesulfonamide (3.36 g) having the following physical properties as colorless crystals (yield 98.2%).
Melting Point: 57° - 58°C (n-hexane)
IR νmax (KBr) cm⁻¹: 3286 (NH), 1328, 1123 (SO₂)
Mass Spectroscopy (C₈H₁₈ClNO₂S) EI: m/z 228 (M⁺ +1), CI: m/z 228 (M⁺ +1)
EI-HRMS (C₈H₁₈ClNO₂S+H⁺)
Calculated: 228.0824, Found: 228.0817
¹H-NMR (CDCl₃) δ: 1.26 (6H, d, J = 6.5 Hz, CH₃×2), 1.54-1.68 (2H, m, ClCH₂CH₂CH₂), 1.79-1.92 (4H, m, ClCH₂CH₂, SCH₂CH₂), 3.01-3.06 (2H, m, SCH₂), 3.57 (2H, t, J = 6.5 Hz, ClCH₂), 3.66 (1H, m, CH), 4.10 (1H, d, J = 7.4 Hz, NH)
Elemental Analysis (C₈H₁₈ClNO₂S)
Calculated: C, 42.19; H, 7.97; N, 6.15, Found: C, 42.40; H, 7.70; N, 6.19

### Example 29 [Preparation of N-Cyclobutyl- 5-chloropentanesulfonamide]

The reaction was carried out in the same manner as in Example 27 except for using cyclobutylamine (2.42 g, 33 mM) in place of cyclopropylamine in Example 27, to give N-cyclobutyl-5-chloropentanesulfonamide (3.42 g) having the following physical properties as colorless crystals (yield 95.0%).
Melting Point: 68° - 70°C (ethyl acetate-ligroin)
IR νmax (KBr) cm⁻¹: 3281 (NH), 1316, 1128 (SO₂)
Mass Spectroscopy (C₉H₁₈ClNO₂S) EI: m/z 240 (M⁺ +1), CI: m/z 240 (M⁺ +1)
EI-HRMS (C₉H₁₈ClNO₂S+H⁺)
Calculated: 240.0824, Found: 240.0821
¹H-NMR (CDCl₃) δ: 1.54-2.03 (10H, m, ClCH₂CH₂CH₂, SCH₂CH₂, CHCH₂CH₂, CHCHH×2), 2.34-2.43 (2H, m, CHCHH×2), 2.96-3.01 (2H, m, SCH₂), 3.56 (2H, t, J = 6.5 Hz, ClCH₂), 3.83-3.98 (1H, m, CH), 4.59 (1H, d, J = 8.8 Hz, NH)
Elemental Analysis (C₉H₁₈ClNO₂S)
Calculated: C, 45.08; H, 7.57; N, 5.84, Found: C, 44.96; H, 7.37; N, 5.70

### Example 30 [Preparation of N-Cyclopentyl- 5-chloropentanesulfonamide]

The reaction was carried out in the same manner as in Example 27 except for using cyclopentylamine (2.81 g, 33 mM) in place of cyclopropylamine in Example 27, to give N-cyclopentyl-5-chloropentanesulfonamide (3.79 g) having the following physical properties as colorless crystals (yield 99.5%).
Melting Point: 84° - 86°C (n-hexane)
IR νmax (KBr) cm⁻¹: 3282 (NH), 1319, 1130 (SO₂)
Mass Spectroscopy (C₁₀H₂₀ClNO₂S) EI: m/z 253 (M⁺)
EI-HRMS (C₁₀H₂₀ClNO₂S)
Calculated: 253.0902, Found: 253.0899
¹H-NMR (CDCl₃) δ: 1.44-1.92 (12H, m, ClCH₂CH₂CH₂, SCH₂CH₂, CHCH₂CH₂×2, CHCHH×2), 1.97-2.09 (2H, m, CHCHH×2), 3.01-3.06 (2H, m, SCH₂), 3.57 (2H, t, J = 6.5 Hz, ClCH₂), 3.72-3.84 (1H, m, CH), 4.30 (1H, d, J = 7.4 Hz, NH)
Elemental Analysis (C₁₀H₂₀ClNO₂S)
Calculated: C, 47.32; H, 7.94; N, 5.52, Found: C, 47.54; H, 7.92; N, 5.23

### Example 31 [Preparation of N-Cyclohexyl- 5-chloropentanesulfonamide]

The reaction was carried out in the same manner as in Example 27 except for using cyclohexylamine (3.27 g, 33 mM) in place of cyclopropylamine in Example 27, to give N-cyclohexyl-5-chloropentanesulfonamide (3.98 g) having the following physical properties as colorless crystals (yield 99.0%).
Melting Point: 78° - 80°C (n-hexane)
IR νmax (KBr) cm⁻¹: 3291 (NH), 1319, 1132 (SO₂)
Mass Spectroscopy (C₁₁H₂₂ClNO₂S) EI: m/z 267 (M⁺)
EI-HRMS (C₁₁H₂₂ClNO₂S)
Calculated: 267.1059, Found: 267.1054
¹H-NMR (CDCl₃) δ: 1.11-2.06 (16H, m, ClCH₂CH₂CH₂, SCH₂CH₂, CHCH₂CH₂×2, CHCH₂CH₂CH₂), 3.01-3.06 (2H, m, SCH₂), 3.23-3.37 (1H, m, CH), 3.57 (2H, t, J = 6.4 Hz, ClCH₂), 4.18 (1H, d, J = 7.6 Hz, NH)
Elemental Analysis (C₁₁H₂₂ClNO₂S)
Calculated: C, 49.33; H, 8.28; N, 5.23, Found: C, 49.29; H, 8.01; N, 4.97

### Example 32 [Preparation of N-(2-Hydroxyethyl)-5-chloropentanesulfonamide]

The reaction was carried out in the same manner as in Example 25 except for using 5-chloropentanesulfonyl chloride (3.08 g, 15 mM) in place of 6-chlorohexanesulfonyl chloride in Example 25, to give a by-product [N,O-bis-(5-chloropetanesulfonyl)ethanolamine] (0.55 g) from a first eluted fraction (yield 18.5%) and a desired compound (2.61 g) from a second eluted fraction as a colorless oily product (yield 75.7%).
IR νmax (neat) cm⁻¹: 3494 (OH), 3295 (NH), 1318, 1144 (SO₂)
Mass Spectroscopy (C₇H₁₆ClNO₃S) EI: m/z 230 (M⁺ +1), CI: m/z 230 (M⁺ +1)
EI-HRMS (C₇H₁₆ClNO₃S+H⁺)
Calculated: 230.0617, Found: 230.0615
¹H-NMR (CDCl₃) δ: 1.54-1.68 (2H, m, ClCH₂CH₂CH₂), 1.79-1.93 (4H, m, ClCH₂CH₂, SCH₂CH₂), 2.36 (1H, brs, OH), 3.09 (2H, t, J = 8.0 Hz, SCH₂), 3.29 (2H, q, J = 4.9 Hz, NCH₂), 3.57 (2H, t, J = 6.5 Hz, ClCH₂), 3.78 (2H, brd, J = 4.5 Hz, OCH₂), 4.97 (1H, m, NH)

### Example 33 [Preparation of N-(3-Hydroxypropyl)-5-chloropentanesulfonamide]

The reaction was carried out in the same manner as in Example 25 except for using 5-chloropentanesulfonyl chloride (3.08 g, 15 mM) in place of 6-chlorohexanesulfonyl chloride, and n-propanolamine (2.48 g, 33 mM) in place of ethanolamine in Example 25, to give a by-product [N,O-bis-(5-chloropetanesulfonyl)-propanolamine] (0.64 g) from a first eluted fraction (yield 20.7%) and a desired compound (2.76 g) from a second eluted fraction as a colorless waxy product (yield 75.4%). The physical properties of the desired compound obtained are as follows.
IR νmax (neat) cm⁻¹: 3500-3200 (OH, NH), 1321, 1135 (SO₂)
Mass Spectroscopy (C₈H₁₈ClNO₃S) EI: m/z 244 (M⁺ +1), CI: m/z 244 (M⁺ +1)
EI-HRMS (C₈H₁₈ClNO₃S+H⁺)
Calculated: 244.0773, Found: 244.0777
¹H-NMR (CDCl₃) δ: 1.54-1.67 (2H, m, ClCH₂CH₂CH₂), 1.78-1.91 (6H, m, ClCH₂CH₂, SCH₂CH₂, NCH₂CH₂), 2.01 (1H, m, OH), 3.05 (2H, t, J = 8.0 Hz, SCH₂), 3.30 (2H, q, J = 6.2 Hz, NCH₂), 3.57 (2H, t, J = 6.4 Hz, ClCH₂), 3.82 (2H, brd, J = 4.9 Hz, OCH₂), 4.84 (1H, m, NH)

### Example 34 [Purification of 5-Bromopentanesulfonyl Chloride]

A crude product (13.32 g) obtained from sodium 5-bromopentanesulfonate (30.37 g, 120 mM) in the same manner as in Example 16, was subjected to silica gel column chromatography and eluted from benzene, to give a desired product (7.33 g) as a pale yellow oily product (total yield 24.5%).

### Example 35 [Preparation of N-Cyclobutyl- 5-bromopentanesulfonamide]

The reaction was carried out in the same manner as in Example 25 except for using cyclobutylamine (2.35 g, 33 mM) in place of cyclopropylamine in Example 27, and 5-bromopentanesulfonyl chloride (crude product) (3.74 g, 15 mM) obtained in Example 16 in place of 6-chlorohexanesulfonyl chloride. The resulting reaction product was subjected to silica gel column chromatography and eluted from ethyl acetate-n-hexane (1:3), to give N-cyclobutyl-5-bromopentanesulfonamide (2.22 g) having the following physical properties as colorless crystals (yield 52.1%).
Melting Point: 71° - 72°C (diethyl ether)
IR νmax (KBr) cm⁻¹: 3284 (NH), 1311, 1128 (SO₂)
Mass Spectroscopy (C₉H₁₈BrNO₂S) EI: m/z 284 (M⁺ +1), CI: m/z 284 (M⁺ +1)
EI-HRMS (C₉H₁₈BrNO₂S+H⁺)
Calculated: 284.0318, Found: 284.0311
¹H-NMR (CDCl₃) δ: 1.57-2.03 (10H, m, BrCH₂CH₂CH₂, SCH₂CH₂, CHCH₂CH₂, CHCHH×2), 2.33-2.43 (2H, m, CHCHH×2), 2.96-3.01 (2H, m, SCH₂), 3.43 (2H, t, J = 6.6 Hz, BrCH₂), 3.85-3.99 (1H, m, CH), 4.49 (1H, d, J = 8.9 Hz, NH)
Elemental Analysis (C₉H₁₈BrNO₂S)
Calculated: C, 38.03; H, 6.38; N, 4.93, Found: C, 38.27; H, 6.28; N, 4.83

### Example 36 [Preparation of N-Cyclobutyl- 5-bromopentanesulfonamide]

In Example 35, the reaction was carried out in the same manner as in Example 35 except for using 5-bromopentanesulfonyl chloride (purified product) (3.74 g, 15 mM) obtained in Example 34, to give a desired product (4.18 g) as colorless crystals (yield 98.1%).

### Example 37 [Preparation of 9-Bromononanesulfonyl Chloride]

In Example 16, the reaction was carried out in the same manner as in Example 16 except for using crude sodium 9-bromononanesulfonate (4.70 g, 15.2 mM) prepared by a process described in *Org. Synth*., Coll. Vol. II, 558 or WO95/19345 Pamphlet and thionyl chloride (9 ml, 123 mM), and a crude product obtained (2.74 g) was subjected to silica gel column chromatography and eluted from benzene-n-hexane (1:2), to give 9-bromononanesulfonyl chloride (1.59 g) having the following physical properties as a pale yellow oily product (yield 34.2%).
IR νmax (neat) cm⁻¹: 1375, 1166 (SO₂)
Mass Spectroscopy (C₉H₁₈BrClO₂S) EI: m/z 305 (M⁺ +1), CI: m/z 305 (M⁺ +1)
EI-HRMS (C₉H₁₈BrClO₂S+H⁺)
Calculated: 304.9977, Found: 304.9985
¹H-NMR (CDCl₃) δ: 1.36-1.55 (10H, m, CH₂×5), 1.83-1.93 (2H, m, BrCH₂CH₂), 2.01-2.11 (2H, m, SCH₂CH₂), 3.43 (2H, t, J = 6.8 Hz, BrCH₂), 3.65-3.71 (2H, m, SCH₂)

### Example 38 [Preparation of 10-Bromodecanesulfonyl Chloride]

In Example 16, the reaction was carried out in the same manner as in Example 16 except for using crude sodium 10-bromodecanesulfonate (30.00 g, 93 mM) prepared by a process described in *Org. Synth*., Coll. Vol. II, 558 or WO95/19345 Pamphlet and thionyl chloride (54 ml, 742 mM), and a crude product obtained (20.55 g) was subjected to silica gel column chromatography and eluted from benzene-n-hexane (1:2), to give 10-bromodecanesulfonyl chloride (5.42 g) having the following physical properties as a pale yellow oily product (yield 18.2%).
IR νmax (neat) cm⁻¹: 1375, 1166 (SO₂)
Mass Spectroscopy (C₁₀H₂₀BrClO₂S) EI: m/z 319 (M⁺ +1), CI: m/z 319 (M⁺ +1)
EI-HRMS (C₁₀H₂₀BrClO₂S+H⁺)
Calculated: 319.0133, Found: 319.0124
¹H-NMR (CDCl₃) δ: 1.34-1.55 (12H, m, CH₂×6), 1.83-1.92 (2H, m, BrCH₂CH₂), 2.01-2.11 (2H, m, SCH₂CH₂), 3.43 (2H, t, J = 6.8 Hz, BrCH₂), 3.65-3.70 (2H, m, SCH₂)

### Example 39 [Preparation of 11-Bromoundecanesulfonyl Chloride]

In Example 16, the reaction was carried out in the same manner as in Example 16 except for using crude sodium 11-bromoundecanesulfonate (8.54 g, 25 mM) prepared by a process described in *Org. Synth*., Coll. Vol. II, 558 or WO95/19345 Pamphlet and thionyl chloride (15 ml, 206 mM), and a crude product obtained (3.79 g) was subjected to silica gel column chromatography and eluted from benzene-n-hexane (1:1), to give 11-bromoundecanesulfonyl chloride (2.43 g) having the following physical properties as a colorless oily product (yield 28.8%).
IR νmax (neat) cm⁻¹: 1377, 1166 (SO₂)
Mass Spectroscopy (C₁₁H₂₂BrClO₂S) EI: m/z 333 (M⁺ +1), CI: m/z 333 (M⁺ +1)
EI-HRMS (C₁₁H₂₂BrClO₂S+H⁺)
Calculated: 333.0289, Found: 333.0275
¹H-NMR (CDCl₃) δ: 1.32-1.55 (14H, m, CH₂×7), 1.84-1.92 (2H, m, BrCH₂CH₂), 2.01-2.12 (2H, m, SCH₂CH₂), 3.43 (2H, t, J = 6.8 Hz, BrCH₂), 3.65-3.70 (2H, m, SCH₂)

### Example 40 [Preparation of 12-Bromododecanesulfonyl Chloride]

In Example 16, the reaction was carried out in the same manner as in Example 16 except for using crude sodium 12-bromododecanesulfonate (32.60 g, 93 mM) prepared by a process described in *Org. Synth*., Coll. Vol. II, 558 or WO95/19345 Pamphlet and thionyl chloride (58 ml, 795 mM), and a crude product obtained (11.06 g) was subjected to silica gel column chromatography and eluted from benzene-n-hexane (1:2), to give 12-bromododecanesulfonyl chloride (5.88 g) having the following physical properties as colorless crystals (yield 18.2%).
Melting Point: 30° - 31°C (n-hexane)
IR νmax (KBr) cm⁻¹: 1360, 1158 (SO₂)
Mass Spectroscopy (C₁₂H₂₄BrClO₂S) EI: m/z 347 (M⁺ +1), CI: m/z 347 (M⁺ +1)
EI-HRMS (C₁₂H₂₄BrClO₂S+H⁺)
Calculated: 347.0446, Found: 347.0441
¹H-NMR (CDCl₃) δ: 1.31-1.54 (16H, m, CH₂×8), 1.83-1.93 (2H, m, BrCH₂CH₂), 2.01-2.12 (2H, m, SCH₂CH₂), 3.43 (2H, t, J = 6.8 Hz, BrCH₂), 3.65-3.70 (2H, m, SCH₂)
Elemental Analysis (C₁₂H₂₄BrClO₂S)
Calculated: C, 41.44; H, 6.96, Found: C, 41.47; H, 7.02

### Example 41 [Preparation of N-Cyclopropyl-9-bromononanesulfonamide]

In Example 27, the reaction was carried out in the same manner as in Example 27 except for using 9-bromononanesulfonyl chloride (1.00 g, 3.2 mM) in place of 5-chloropentanesulfonyl chloride, and cyclopropylamine (0.41 g, 7.0 mM), to give N-cyclopropyl-9-bromononanesulfonamide (1.02 g) having the following physical properties as colorless crystals (yield 97.9%).
Melting Point: 60° - 62°C (chloroform-n-hexane)
IR νmax (KBr) cm⁻¹: 3274 (NH), 1315, 1135 (SO₂)
Mass Spectroscopy (C₁₂H₂₄BrNO₂S) EI: m/z 326 (M⁺ +1), CI: m/z 326 (M⁺ +1)
EI-HRMS (C₁₂H₂₄BrNO₂S+H⁺)
Calculated: 326.0788, Found: 326.0797
¹H-NMR (CDCl₃) δ: 0.67-0.76 (4H, m, CHCH₂×2), 1.34-1.49 (10H, m, CH₂×5), 1.77-1.92 (4H, m, BrCH₂CH₂, SCH₂CH₂), 2.57-2.63 (1H, m, CH), 3.06-3.11 (2H, m, SCH₂), 3.42 (2H, t, J = 6.8 Hz, BrCH₂), 4.62 (1H, brs, NH)
Elemental Analysis (C₁₂H₂₄BrNO₂S)
Calculated: C, 44.17; H, 7.41; N, 4.29, Found: C, 44.30; H, 7.61; N, 4.53

### Example 42 [Preparation of N-Cyclopropyl-10-bromodecanesulfonamide]

The reaction was carried out in the same manner as in Example 27 except for using 10-bromodecanesulfonyl chloride (450 mg, 1.41 mM) in place of 5-chloropentanesulfonyl chloride in Example 27, and cyclopropylamine (177 mg, 3.10 mM), to give N-cyclopropyl-10-bromodecanesulfonamide (467 mg) having the following physical properties as colorless crystals (yield 97.5%).
Melting Point: 57° - 60°C (chloroform-n-hexane)
IR νmax (KBr) cm⁻¹: 3273 (NH), 1317, 1135 (SO₂)
Mass Spectroscopy (C₁₃H₂₆BrNO₂S) EI: m/z 340 (M⁺ +1), CI: m/z 340 (M⁺ +1)
EI-HRMS (C₁₃H₂₆BrNO₂S+H⁺)
Calculated: 340.0945, Found: 340.0938
¹H-NMR (CDCl₃) δ: 0.67-0.79 (4H, m, CHCH₂×2), 1.32-1.48 (12H, m, CH₂×6), 1.79-1.93 (4H, m, BrCH₂CH₂, SCH₂CH₂), 2.56-2.62 (1H, m, CH), 3.06-3.11 (2H, m, SCH₂), 3.42 (2H, t, J = 6.8 Hz, BrCH₂), 4.60 (1H, brs, NH)
Elemental Analysis (C₁₃H₂₆BrNO₂S)
Calculated: C, 45.88; H, 7.70; N, 4.12, Found: C, 46.03; H, 7.59; N, 4.27

### Example 43 [Preparation of N-Cyclopropyl-11-bromoundecanesulfonamide]

The reaction was carried out in the same manner as in Example 27 except for using 11-bromoundecanesulfonyl chloride (2.29 g, 6.85 mM) in place of 5-chloropentanesulfonyl chloride in Example 27, and cyclopropylamine (860 mg, 15.1 mM), to give N-cyclopropyl-11-bromoundecanesulfonamide (2.33 g) having the following physical properties as colorless crystals (yield 96.3%).
Melting Point: 71° - 73°C (chloroform-n-hexane)
IR νmax (KBr) cm⁻¹: 3274 (NH), 1317, 1135 (SO₂)
Mass Spectroscopy (C₁₄H₂₈BrNO₂S) EI: m/z 354 (M⁺ +1), CI: m/z 354 (M⁺ +1)
EI-HRMS (C₁₄H₂₈BrNO₂S+H⁺)
Calculated: 354.1101, Found: 354.1092
¹H-NMR (CDCl₃) δ: 0.65-0.79 (4H, m, CHCH₂×2), 1.31-1.48 (14H, m, CH₂×7), 1.74-1.92 (4H, m, BrCH₂CH₂, SCH₂CH₂), 2.57-2.62 (1H, m, CH), 3.06-3.11 (2H, m, SCH₂), 3.42 (2H, t, J = 6.8 Hz, BrCH₂), 4.61 (1H, brs, NH)

### Example 44 [Preparation of N-Cyclopropyl- 12-bromododecanesulfonamide]

The reaction was carried out in the same manner as in Example 27 except for using 12-bromododecanesulfonyl chloride (2.92 g, 8.4 mM) in place of 5-chloropentanesulfonyl chloride in Example 27, and cyclopropylamine (1.08 g, 18.5 mM), to give N-cyclopropyl-12-bromododecanesulfonamide (2.99 g) having the following physical properties as colorless crystals (yield 96.8%).
Melting Point: 71° - 73°C (n-hexane)
IR νmax (KBr) cm⁻¹: 3274 (NH), 1318, 1135 (SO₂)
Mass Spectroscopy (C₁₅H₃₀BrNO₂S) EI: m/z 368 (M⁺ +1), CI: m/z 368 (M⁺ +1)
EI-HRMS (C₁₅H₃₀BrNO₂S+H⁺)
Calculated: 368.1257, Found: 368.1261
¹H-NMR (CDCl₃) δ: 0.66-0.79 (4H, m, CHCH₂×2), 1.30-1.50 (16H, m, CH₂×8), 1.74-1.92 (4H, m, BrCH₂CH₂, SCH₂CH₂), 2.57-2.63 (1H, m, CH), 3.06-3.11 (2H, m, SCH₂), 3.43 (2H, t, J = 6.8 Hz, BrCH₂), 4.58 (1H, brs, NH)
Elemental Analysis (C₁₅H₃₀BrNO₂S)
Calculated: C, 48.90; H, 8.21; N, 3.80, Found: C, 49.18; H, 8.36; N, 4.08

### Example 45 [Preparation of N-(2-Hydroxyethyl)-6-chlorohexanesulfonamide]

In Example 25, the reaction was carried out in the same manner as in Example 25 except for using ethanolamine (3.02 g, 49.5 mM), to give a desired product (3.21 g) as a colorless oily product (yield 87.7%).

### Example 46 [Preparation of N-(3-Hydroxypropyl)-6-chlorohexanesulfonamide]

The reaction was carried out in the same manner as in Example 25 except for using n-propanolamine (3.72 g, 49.5 mM) in place of the ethanolamine in Example 25, to give a desired product (3.36 g) as a colorless waxy product (yield 86.8%).

### Example 47 [Preparation of N-(2-Hydroxyethyl)-5-chloropentanesulfonamide]

The reaction was carried out in the same manner as in Example 25 except for using 5-chloropentanesulfonyl chloride (3.08 g, 15 mM) in place of 6-chlorohexanesulfonyl chloride in Example 25, and ethanolamine (3.02 g, 49.5 mM), to give a desired product (2.92 g) as a colorless oily product (yield 84.6%).

### Example 48 [Preparation of N-(3-Hydroxypropyl)-5-chloropentanesulfonamide]

The reaction was carried out in the same manner as in Example 25 except for using 5-chloropentanesulfonyl chloride (3.08 g, 15 mM) in place of 6-chlorohexanesulfonyl chloride, and n-propanolamine (3.72 g, 49.5 mM) in place of ethanolamine in Example 25, to give a desired product (3.12 g) as a colorless waxy product (yield 85.2%).

### Example 49 [Preparation of N-(2-Hydroxyethyl)-6-chlorohexanesulfonamide]

Sodium hydroxide (0.60 g, 15 mM) was added to a suspension of diethyl ether (15 ml) and ethanolamine (1.37 g, 22 mM). A diethyl ether solution (15 ml) of 6-chlorohexanesulfonyl chloride (3.29 g, 15 mM) was added dropwise thereto under ice-cooling. Thereafter, the mixture was stirred at room temperature. After the reaction was completed, the reaction product obtained was extracted with ethyl acetate (50 ml). The extract obtained was dried over anhydrous magnesium sulfate. Subsequently, the solvent was removed by evaporation *in vacuo*, and the crude product was subjected to silica gel column chromatography and eluted from a mixed liquid of chloroform-methanol (20:1), to give a desired product (2.94 g) as a colorless oily product (yield 80.4 %).

The results mentioned above show that halogenoalkylsulfonamide derivatives can be obtained efficiently in accordance with the methods of the examples.

### EQUIVALENTS

Those skilled in the art will recognize, or be able to ascertain using simple routine experimentation, many equivalents to the specific embodiments of the invention described in the present specification. Such equivalents are intended to be encompassed in the scope of the present invention as described in the following claims.

### INDUSTRIAL APPLICABILITY

According to the method of the present invention, there can be economically and industrially advantageously prepared a halogenoalkylsulfonamide derivative which is an intermediate for preparing a diazacycloalkanealkylsulfonamide derivative useful as an antiallergic agent and the like.

## Claims

1. A process for preparing a halogenoalkylsulfonamide derivative represented by the general formula (Ia): 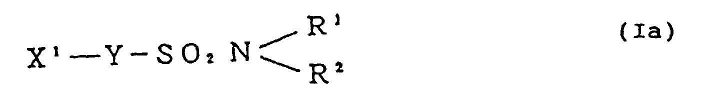 wherein R¹ is a hydroxyalkyl group having 1 to 4 carbon atoms or a cycloalkyl group having 3 to 8 carbon atoms; R² is hydrogen atom, a straight or branched chain alkyl group having 1 to 6 carbon atoms, a hydroxyalkyl group having 1 to 4 carbon atoms, a cycloalkyl group having 3 to 8 carbon atoms, or a phenyl group which may have on its phenyl ring 1 to 3 substituents selected from the group consisting of an alkyl group having 1 to 4 carbon atoms, an alkoxy group having 1 to 4 carbon atoms, a halogen atom, hydroxyl group, trifluoromethyl group, nitro group and amino group; X¹ is chlorine atom or bromine atom; and Y is an alkylene group having 5 to 12 carbon atoms,
characterized by reacting a halogenoalkylsulfonyl chloride represented by the general formula (II):
X¹-Y-SO₂Cl (II)
wherein X¹ and Y are as defined above, with
an amine represented by the general formula (III): 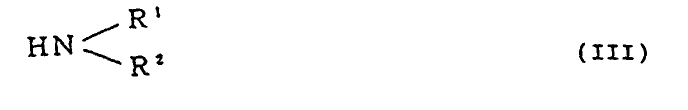 wherein R¹ and R² are as defined above.

2. The process for preparing a halogenoalkylsulfonamide derivative as claimed in claim 1, wherein the halogenoalkylsulfonyl chloride is reacted with the amine in the presence of an inorganic base.

3. The process for preparing a halogenoalkylsulfonamide derivative as claimed in claim 2, wherein the inorganic base is an alkali metal hydroxide.

4. The process for preparing a halogenoalkylsulfonamide derivative as claimed in claim 2, wherein the amount of the inorganic base is 1 to 1.5 mol per one mol of the halogenoalkylsulfonyl chloride.

5. The process for preparing a halogenoalkylsulfonamide derivative as claimed in claim 4, wherein the amount of the amine is 1 to 3 mol per one mol of the halogenoalkylsulfonyl chloride.

6. The process for preparing a halogenoalkylsulfonamide derivative as claimed in claim 1, wherein R¹ is a hydroxyalkyl group having 1 to 4 carbon atoms, and R² is hydrogen atom in the amine represented by the general formula (III).

7. The process for preparing a halogenoalkylsulfonamide derivative as claimed in claim 1, wherein R¹ is a cycloalkyl group having 3 to 8 carbon atoms, and R² is hydrogen atom in the amine represented by the general formula (III).

8. A halogenoalkylsulfonyl chloride represented by the general formula (IIa):
Br-Y-SO₂Cl (IIa)
wherein Y is an alkylene group having 5 to 8 carbon atoms.

9. A process for preparing a halogenoalkylsulfonyl chloride represented by the general formula (IIa):
Br-Y-SO₂Cl (IIa)
wherein Y is an alkylene group having 5 to 8 carbon atoms, characterized by reacting a sodium bromoalkylsulfonate represented by the general formula (IV):
Br-Y-SO₃Na (IV)
wherein Y is as defined above, with
a chlorinating agent.
